# EUROPEAN PATENT APPLICATION

(11) **EP 1 118 267 A1**
(43) Date of publication of application: **25.07.2001**
(21) Application number: 00101176.6
(22) Date of filing: 21.01.2000
(51) Int. Cl.: A01K 45/00, A01K 43/00, G01N 33/08

(54) **A method and system for verification of fertilization of poultry eggs**

(71) Applicant: Cohen, Michael, 90912 Mobile Post North Yehuda (IL)
(72) Inventor: Cohen, Michael, 90912 Mobile Post North Yehuda (IL)
(74) Representative: Klunker . Schmitt-Nilson . Hirsch

(57) **Abstract**

The invention provides a method for verification of the presence of a living embryo in an incubating poultry egg, comprising the steps of causing at least two electrodes (4,4',4'') to make conductive contact with the outside surface of the shell of an egg; amplifying any of the analog signals received via two of the at least two electrodes; analyzing the signals after amplification, using a program in a microprocessor to establish whether the signals originated in the cardiac activity of the embryo, and providing information confirming or negating the presence of a living embryo in the egg. The invention also provides a system for carrying out the method.

## Description

### Field of the Invention

The present invention relates to a method and a system for verification of the presence of living poultry embryos in incubating poultry eggs.

### Background of the Invention

About 10% of all eggs reaching chick hatcheries are non-fertile and, for both economic and hygienic reasons, the hatcheries are interested in eliminating these eggs at the earliest possible opportunity. The conventional process used to this end is known as "candling," in which, on about the 10th day of incubation, an experienced operator in a darkened room manually passes a strong light source from egg to egg, looking for a network of tiny blood vessels in each now translucent egg, which indicate a developing embryo. Eggs lacking this network are immediately discarded, as they obviously will not end up as chicks; nor are they, at this stage, any longer fit for human consumption.

The time-honored method of candling has several serious disadvantages: it requires trained manpower (large hatcheries incubate tens of thousands of eggs at any given time), yet even the most experienced operators will fail to correctly diagnose one out of every ten eggs; they will either fail to recognize the capillaries, or they will pass as fertile an egg in which the capillaries are still discernible, thus giving the appearance of a properly broodable egg, while the embryo has in fact died.

In recent years, attempts have been made to increase the low efficiency, and reduce the high demands for operator skill, of the candling method by introducing devices built around optical systems that, to some extent, could also be automated. Yet while these improved candling devices could, with some reliability, differentiate between fertilized and non-fertilized chicken eggs, they failed tQ distinguish between fertilized eggs containing a live embryo and eggs which, although still displaying the capillaries, contain embryos that have died.

What is more, these optical devices are altogether unsuitable for the checking of turkey eggs, the shells of which are thicker and far less translucent than those of chicken eggs.

### Summary of the Invention

It is thus one of the objects of the present invention to provide a method for verification of the presence of living poultry embryos in incubating eggs, which method is not based on the expertise of human operators, has a much smaller error margin, and can be largely automated.

It is a further object of the present invention to provide a method that will pick out for discarding not only non-fertilized eggs, but also originally fertilized eggs in which the embryo has died.

It is a still further object of the invention to provide a method for handling turkey eggs, the shells of which are much thicker and far less translucent than those of chicken eggs.

According to the invention, these objectives are achieved by providing a method for verification of the presence of a living embryo in an incubating poultry egg, comprising the steps of causing at least two electrodes to make conductive contact with the outside surface of the shell of said egg; amplifying any of the analog signals received via two of said at least two electrodes; analyzing said signals after amplification, using a program in a microprocessor to establish whether said signals originated in the cardiac activity of said embryo, and providing information confirming or negating the presence of a living embryo in said egg.

The present invention further provides a system for verification of the presence of a living embryo in incubating poultry eggs, comprising an array of detector heads, each provided with at least two electrodes adapted to make contact with the surface of the shell of one of said eggs; microprocessor means responsive to signals received by said electrodes and adapted to establish the presence or absence of signals originating in the cardiac activity of a living embryo, and means controlled by said microprocessor to facilitate separation of the eggs found lacking such a presence from those in which such a presence has been confirmed.

The method according to the invention is based on the fact that the cardiac activity of the embryo produces characteristic electrical signals as early as at the end of the third day of incubation. By amplifying these signals, picked up by non-invasive electrodes that leave the eggshell intact, and analyzing them after separating them from the inevitable noise superposed on them, proof can be obtained not only that the egg has been fertilized, but also that it contains a live embryo. Conversely, the absence of such signals signifies a non-fertilized egg or a dead embryo in an originally fertilized egg.

The invention will now be described in connection with certain preferred embodiments with reference to the following illustrative figures so that it may be more fully understood.

With specific reference now to the figures in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

### Brief Description of the Drawings

- Fig. 1: is a general block diagram of the method according to the present invention;
- Fig. 2: is a more detailed flow diagram illustrating the method according to the invention;
- Fig. 3: is a cross-sectional elevational view along plane III-III of Fig. 4, of a detector head according to the present invention;
- Fig. 4: is a top view of the three-pronged electrode holders of the detector head of Fig. 3;
- Fig. 5: is a schematic top view of the work station of the system according to the invention;
- Fig. 6: i is a top view of the egg tray used by the system;
- Fig. 7: is a schematic side view of a testing unit of the system;
- Fig. 8: is a schematic side view of a unit for separating the eggs found to be non-incubatable, and
- Fig. 9: is a characteristic curve of embryo heartbeats .

### Detailed Description of Preferred Embodiments

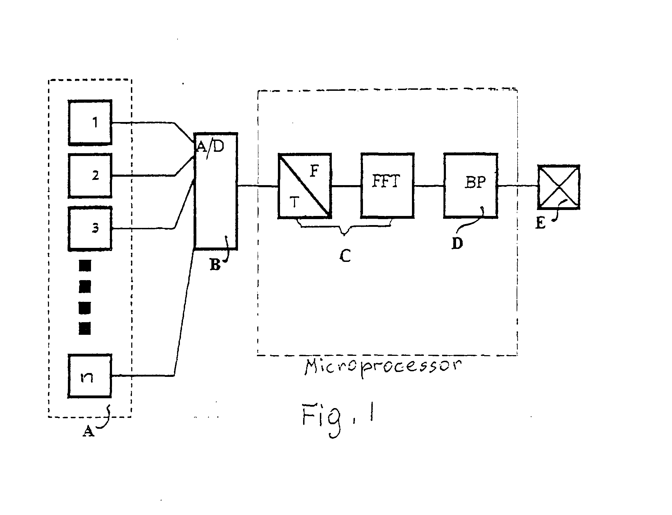

Referring now to the drawings, there is seen in Fig. 1 a block diagram in which block *A* is a data acquisition block comprising a plurality of *n* detector heads provided with electrodes that can be brought into contact with the eggs to be tested. The actual configuration of the detector heads will be shown and explained further below.

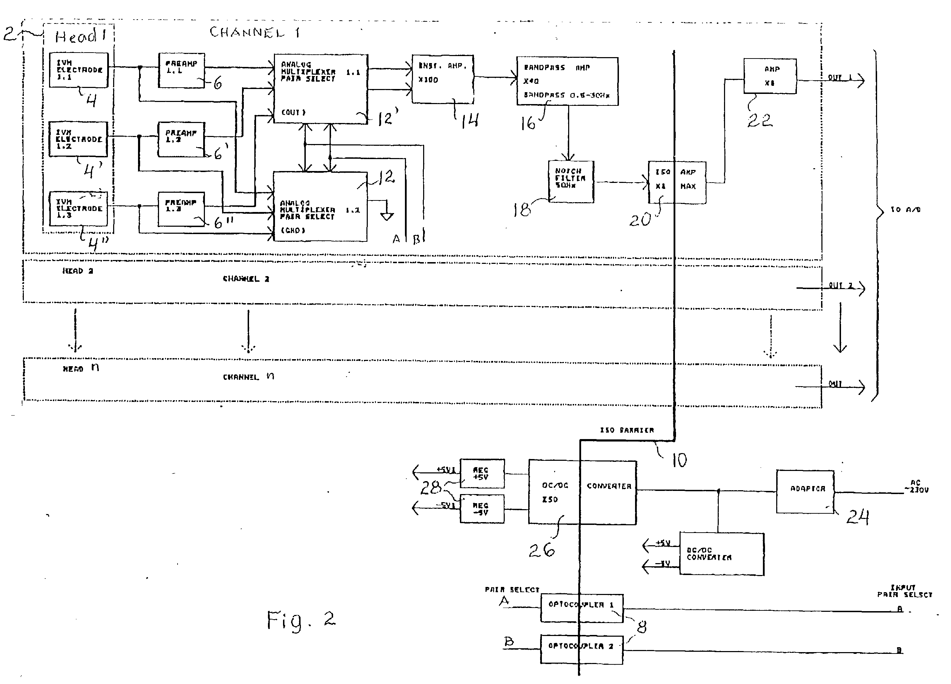

Each electrode of a detector head is provided with its own preamplifier. All the electronic components belonging to block *A* and shown in greater detail in Fig. 2 are accommodated within a Faraday-cage type space to reduce noise, *i.e.,* to improve the signal-to-noise ratio.

Block *B* is the analog-to-digital (A/D) conversion block and, in the present embodiment, uses an ADLINK A/D card. Block *B* is the connecting link to a microprocessor setup, the following blocks of which are controlled by dedicated software:

Block *C* performs a fast Fourier transform, determining the frequency domain and calculating the signal spectrum;

Block *D* performs the required analysis. Experience has shown the pulse of an embryo of 20 days to be about 3 Hz. A bandpass filter of 2 Hz - 4 Hz is advantageously used, with the spectrum power being calculated within this range.

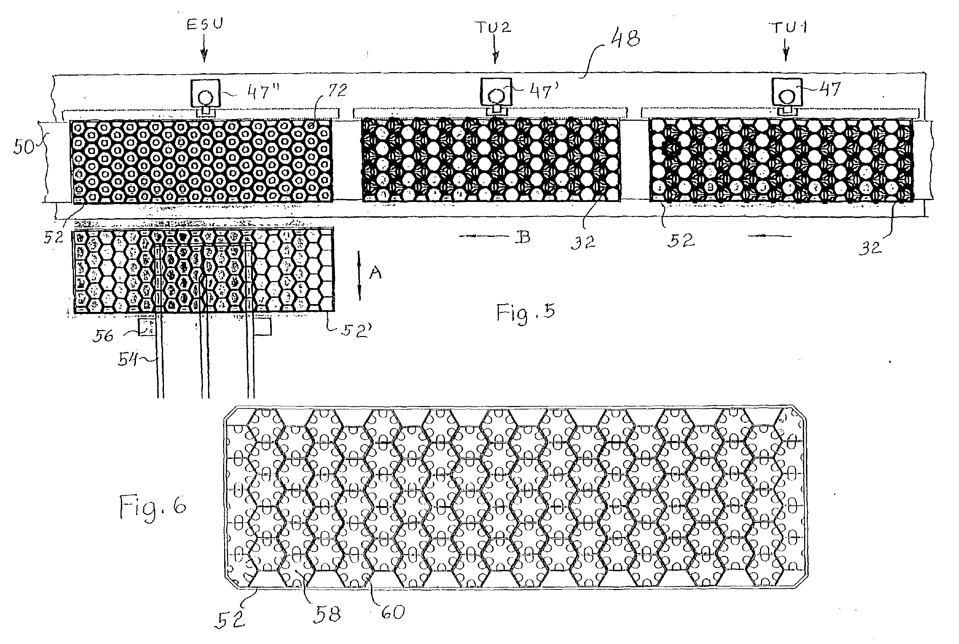

Block *E* represents a monitor, graphically indicating those eggs in a tray (see Fig. 5) that are to be discarded, being either non-fertilized or containing a dead embryo.

The flow diagram of Fig. 2 represents a plurality of *n* channels, of which only channel 1 is fully detailed, it being understood that channels 2*-n* are identical to channel 1. Each channel comprises one detector head 2, to be fully described further below, each head 2 being provided with three electrodes 4, 4', 4", the characteristic properties of which will be explained in conjunction with the description of detector heads 2.

Each of the electrodes 4, 4', 4" is provided with its own preamplifier 6, 6', 6", respectively, which serves to overcome the ohmic resistance of the egg shell, estimated to be of an order of magnitude of several MΩ, to facilitate the transfer of the primary signal to the next amplifier, using standard wire leads.

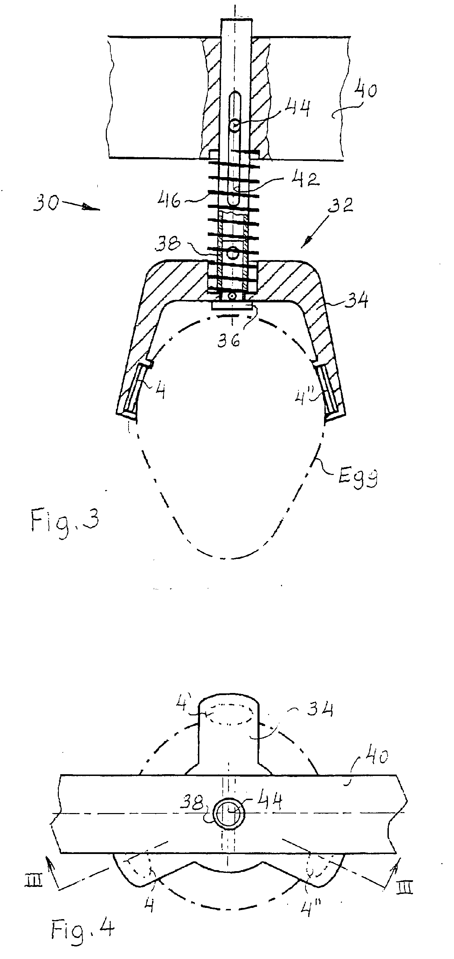

Although the acquisition of a signal indicating the cardiac activity of the embryo can be effected by means of only two electrodes (of which one is grounded), the present invention uses three electrodes because of the fact that the strength of the signal obtained is clearly affected by the distance of the electrodes from the embryonic heart. Now, since the latter is scarcely ever located precisely on the longitudinal axis of the egg, straight lines from the points of contact of diametrically opposite electrodes to the embryonic heart would rarely, if ever, constitute the optimum, *i.e*., shortest, path. For this reason, the present invention provides three electrodes 4, 4', 4", angularly spaced apart by 120° (see Fig. 4), from which, by permutational signal sampling, that pair of electrodes is selected which yields the strongest signals. This selection is controlled by the microprocessor through leads *A, B* via optocouplers 8 and effected by analog multiplexers electrodes '12, 12', which act as electronic switches, with multiplexer 12 switching between the grounded (reference) electrodes and multiplexer 12' between the non-grounded output electrodes.

The signal from the selected pair of electrodes is fed to instrument amplifier 14, where it is amplified, *e.g*., × 100 and led to bandpass amplifier 16 for an *e.g.*, × 40 amplification of the signal and the filtering out of frequencies lower than 0.5 Hz and higher than 30 Hz, which are not required for the purpose of the system.

Further processing of the signal is performed by notch filter 18, which filters out the 50 Hz noise produced by the 220 V A.C. mains.

The signal leaves the Faraday-cage environment via ISO amplifier *X*1, which cleans it of wireless noise before it is fed to final amplifier X6 and led to the A/D block represented in Fig. 1.

The lower part of Fig. 2 represents the mains connection and the electrode pair selection control lines *A, B* from the microprocessor to analog multiplexers 12, 12'. Adaptor 24 reduces the mains voltage from 230 V to 5 V and rectifies the output voltage. Component 26 is an electrolytic ISO DC/DC converter without direct connection, preventing noise and short-circuiting in case one of the circuits is shorted. Power supply 28 serves those circuits that are noise-protected.

Components 8, as already mentioned, are optocouplers bridging screening barrier 10 and transmitting the electrode selection commands from the microprocessor.

Not shown in Fig. 2 are the microprocessor complex and the indicator block *E* of Fig. 1. The information obtained from the signal analysis in block *D* and stored in the memory bank of the microprocessor is used by the system to identify the eggs not containing a living embryo and to facilitate the discarding thereof, as will be described further below.

Apart from the electronics discussed so far, the system according to the present invention comprises a plurality of detector heads equipped with electrodes; slides carrying these heads and movable vertically up and down; trays accommodating, in well-defined arrays, the eggs to be examined, and a slide carrying suction cups for removing the eggs found not to contain a living embryo. These components of the system will now be described.

The detector head 30, illustrated in Figs. 3 and 4, is seen to comprise a three-pronged electrode holder 32 whose arms 34 (Fig. 3) have been swung into the paper plane for simplicity. To the end of each arm 34 there is attached one of electrodes 4, 4', 4". These electrodes advantageously consist of a silver-silver chloride alloy and have a thickness of about 0.6 mm. Their electrical characteristics are:

| | |
|---|---|
| DC offset voltage (bios potential) | 180µV |
| drift (at constant temperature) | 25µV/h |
| noise (0.1-1 kHz, with 60 Hz filtered out) | 1µV P-P |
| polarization (counter EMF) | 2-4 µV at 0.1 µA. |

These electrodes are intended to receive the weak electrical signals originating in the active heart of the living embryo.

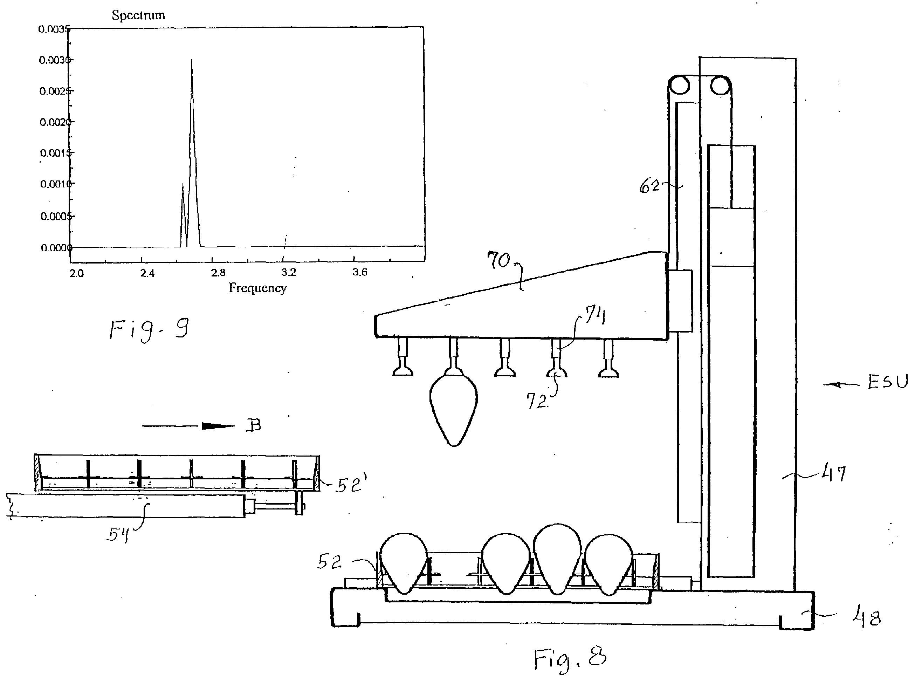

Fixedly attached to holder 32 is a plug 36, over which is slipped, and to which is pinned, a tubular member 38. The latter is slidingly guided in a crossbeam 40 which, in this particular embodiment, carries another four detector heads 30 (see Fig. 7), *i.e*., five heads altogether.

An elongated slot 42 passing through tubular member 38 cooperates with a pin 44 seated in crossbeam 40 and permits detector head 30 one degree of freedom in translation only, *i.e.,* head 30 can move up and down, but it cannot rotate about the axis of tubular member 38. Pin 44 also limits the up-and-down stroke of detector head 30.

A helical spring 46 provides the contact pressure required for electrodes 4, 4', 4" to function properly. There are ten crossbeams 40 to a testing unit, as will become apparent in conjunction with Figs. 5 and 7. When beams 40 are lowered towards the eggs, spring 46 is deformed in compression as soon as electrodes 4, 4', 4" touch the eggshell, providing adequate pressure without endangering the eggshell. The eggs to be assayed are arrayed in special trays, to be discussed further below, which trays ensure that half of the eggs are in alignment with the array of detector heads 30.

Fig. 4 is a top view of a detector head 30, showing the arms 34 of three-pronged electrode holder 32, which arms are angularly spaced by 120°. As can be learned from Fig. 7, adjacent detector heads 30 are mutually offset angularly by 60°, so that their arms 34 will not interfere with one another.

Fig. 5 is a schematic top view of the work stations of the system, from which, for greater clarity, the respective slides of the testing units *TU*1, *TU*2 and the egg separating unit *ESU* have been removed, showing only uprights 47, 47', 47", along which these slides can move.

There is seen a bench or table 48, along which moves a conveyor belt 50 carrying three trays 52 of the type shown in greater detail in Fig. 6. A fourth, empty tray 52', the purpose of which will become apparent further below, is located adjacent to and above the plane of conveyor belt 50, as seen in Fig. 8. Trays 52 of this embodiment are designed to accommodate 100 eggs each, and those trays that are located on conveyor 50 (Fig. 5) are all full of eggs. The markings on every second column of eggs in trays 52 of *TU*1 and *TU*2 symbolize electrode holders 32 (Fig. 3), being parts of detector heads 30, the rest of which have been removed with the respective slides, as stated above. It can be seen that electrode holders 32 of testing unit *TU*1 make contact only with the eggs in every odd-numbered column of eggs (counting from the right), while holders of testing unit *TU2* make contact only with the eggs in every even-numbered column of eggs. The small circles within the eggs of tray 52 of the egg-separating unit *ESU* symbolize suction cups (see also Fig. 8), by means of which, in a sequence to be explained further below, the non-incubatable eggs are separated from the incubatable ones. The empty tray 52' is used to remove the non-incubatable eggs, in a manner to be described in conjunction with Fig. 8.

Telescoping beams 54 mounted on base plates 56 support the egg removal tray 52' which, by mechanical or pneumatic means, is capable of moving in the direction of double arrow *A*. A more detailed description of this movement will also be given in conjunction with Fig. 8.

Fig. 6 is a top view of egg tray 52, which is seen to consist, in this embodiment, of 100 largely hexagonal cells 58, arranged in a honeycomb pattern in 20 columns having 5 cells each, each column being offset from its adjacent column by half the pitch, as dictated by the pattern. Each cell 58 is provided with a number of radially directed lugs 60 which, in cooperation with the walls of each cell 58, serve to hold the eggs to be tested in a substantially vertical position (see Fig. 7).

Fig. 7 is a side elevation of testing units *TU*1 and *TU*2. Seen is bench 48, upright 47 with its guideway 62, along which slide 64 can move, balanced by counterweight 66. Slide 64 carries detector heads 30, as well as the Faraday cage 68 which accommodates all of the electronic components belonging to block A in Fig. 1 and detailed in Fig. 2. Slide 64, located about egg tray 52, can be raised and lowered either manually or electrically. Fig. 7 shows that the system according to the invention can simultaneously process eggs of different sizes, as springs 46 will compensate for any such differences. Not shown in Fig. 7 is conveyor belt 50 of Fig. 5. The actual testing procedure and sequence will be explained further below.

Fig. 8 is a side elevation of the egg separating unit *ESU*, briefly discussed above in conjunction with Fig. 5. Apart from bench 48, upright 47 and tray 52, slide 70 is seen riding along guideway 62. Slide 70, like slide 64, is raisable and lowerable either manually or electrically. Slide 70 carries 100 spring-loaded suction cups 72, telescopingly mounted in stems 74. As indicated in Fig. 5, suction cups 72 are aligned with the centers of cells 58, *i.e*., with the eggs seated in these cells. Each cup 72 is connected to a vacuum manifold via its own solenoid valve (not shown), controlled by the microprocessor of the system. The egg seen to be suspended from one of cups 72 was found non-incubatable by testing unit *TU*1 or *TU*2, and, the relevant tray 52 having reached the egg separating unit *ESU*, slide 70 is lowered for cups 72 to make contact with the eggs, and a command is issued by the microprocessor to the solenoid valve of that cup which is in contact with the non-incubatable egg, which is then lifted off tray 52 when slide 70 is raised. Egg removal tray 52' is then pushed forward in the direction of arrow *B* until it is aligned with lower tray 52. Slide 70 is now lowered until the rejected egg enters the appropriate cell in tray 52', at which moment a command from the microprocessor breaks the vacuum, causing the egg to be released by suction cup 72, after which tray 52' is withdrawn and the non-incubatable eggs are discarded.

The sequence preceding the above-described egg separation and disposal is as follows:

Assuming conveyor belt 50 to be empty of trays 52, as is the case, *e.g*., at the beginning of the working day, a full tray 52 is placed on conveyor belt 50 in front of testing unit *TU*1. Slide 64 (Fig. 7) is now lowered until detector heads 30 make contact with the eggs in columns 1, 3, 5 ... 19, after which the electronic part of the system described above is switched on and, after some seconds, has determined which eggs in the array of odd-numbered columns are non-incubatable. The results of the above-described analysis are stored in the memory bank of the microprocessor and conveyor belt 50 moves one step, after which the station in front of *TU*1 is vacant and the station in front of *TU*2 is occupied by tray 52, in which the eggs in the odd-numbered columns have just been tested. The station in front of *ESU* is still vacant.

A second full tray 52 is now placed in front of *TU*1 and the eggs therein are analyzed, with the results again being stored. At the same time, detector heads 30 of slide 64 of *TU*2 are brought into contact with the eggs in columns 2, 4, 6 ... 20, these eggs are analyzed and the results stored, and conveyor belt 50 again advances by one step, after which the station in front of *TU*1 is vacant, the station in front of *TU2* is occupied by the second tray 52, the eggs of the odd-numbered columns of which have just been tested, and the station in front of *ESU* is now occupied by the first tray, whose full complement of eggs, *i.e*., all odd-numbered and all even-numbered columns of eggs, have been analyzed in the previous two steps. At this point, the microprocessor retrieves the stored results of the analyses, initiating the above-described egg separation and discarding sequence. Simultaneously, a full tray 52 is placed on conveyor belt 50 in front of *TU*1 and the cycle repeats itself.

The present method can be utilized not only to determine fertilization of eggs, but also to distinguish between the sexes of the embryos. Referring to Fig. 9, there is shown a frequency vs amplitude curve of embryo heart beat pulses measured by the method and apparatus of the present invention. It can be clearly seen that beats measured at about 2.7 Hz, which is about 160 beats per minute, exhibit two peaks, at a difference of between about 0.5-0.8 Hz, indicating pulses of different sexes of the embryos.

It will be evident to those skilled in the art that the invention is not limited to the details of the foregoing illustrated embodiments and that the present invention may be embodied in other specific forms without departing from the spirit or essential attributes thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. A method for verification of the presence of a living embryo in an incubating poultry egg, comprising the steps of:
causing at least two electrodes to make conductive contact with the outside surface of the shell of said egg;
amplifying any of the analog signals received via two of said at least two electrodes;
analyzing said signals after amplification, using a program in a microprocessor to establish whether said signals originated in the cardiac activity of said embryo, and
providing information confirming or negating the presence of a living embryo in said egg.

2. The method as claimed in claim 1, further comprising the step of selecting from said at least two electrodes that pair of two electrodes which yields the strongest signal.

3. The method as claimed in claim 1, further comprising the step of filtering said amplified signal to reduce extraneous noise superposed on said signal.

4. The method as claimed in claim 1, further comprising the step of converting said amplified and filtered analog signal into a digital signal to be analyzed by said microprocessor program.

5. The method as claimed in claim 1, further comprising the step of storing said information in the memory bank of said microprocessor.

6. The method as claimed in claim 1, further comprising the step of retrieving said information from said memory bank and using it to facilitate separation of the eggs found lacking the presence of a living embryo from those in which such a presence has been confirmed.

7. The method as claimed in claim 1, further comprising the step of turning said information into a visual display.

8. A system for verification of the presence of living embryos in incubating poultry eggs, comprising:
an array of detector heads, each provided with at least two electrodes adapted to make contact with the surface of the shell of one of said eggs;
microprocessor means responsive to signals received by said electrodes and adapted to establish the presence or absence of signals originating in the cardiac activity of a living embryo, and
means controlled by said microprocessor to facilitate separation of the eggs found lacking such a presence from those in which such a presence has been confirmed.

9. The system as claimed in claim 8, further comprising filter means for reducing extraneous noise superposed on said signals.

10. The system as claimed in claim 8, further comprising an egg tray disposable in a defined position on a table, said egg tray being constituted by an array of cells alignable with said array of detector heads and open at least at the top, into which cells the eggs to be examined are insertable and in which they rest, with their large ends pointing upwards.

11. The system as claimed in claim 8, wherein each of said detector heads is provided with three electrodes angularly spaced by 120°, from which three electrodes that pair of two electrodes which yields the strongest signal is selected by an analog multiplexer means controlled by said microprocessor, through a process of signal sampling.

12. The system as claimed in claim 8, wherein said detector heads are mounted on a vertically translatable slide having a resting position wherein said electrodes are remote from said eggs, and a working position wherein said electrodes are in conductive contact with the shells of said eggs.

13. The system as claimed in claims 8 and 10, further comprising an array of suction cups congruent with said array of cells and mounted on a vertically translatable slide having a resting position wherein said suction cups are remote from said eggs and a working position wherein said suction cups are in contact with said eggs.

14. The system as claimed in claim 13, wherein said suction cups are controlled by said microprocessor to the effect that eggs found to be lacking the presence of a living embryo are lifted off said tray when said slide rises from said working position to said resting position.

15. The system as claimed in claim 14, further comprising means for removing and discarding the eggs lifted off said tray.

16. A method for verification of the presence of a living embryo in an incubating poultry egg, substantially as hereinbefore described and with reference to the accompanying drawings.

17. A system for verification of the presence of living embryos in incubating poultry eggs, substantially as hereinbefore described and with reference to the accompanying drawings.
